# DEMANDE DE BREVET EUROPEEN

(11) **EP 3 766 593 A1**
(43) Date de publication de la demande: **20.01.2021**
(21) Numéro de dépôt: 20185486.6
(22) Date de dépôt: 13.07.2020
(51) Int. Cl.: B09B 3/00, B09B 5/00, C12M 1/107, C12M 3/00, C12M 1/00, C12M 1/33

(54) **PROCÉDÉ DE MÉTHANISATION**

(30) Priorité: 17.07.2019 FR 1908056
(71) Demandeur: La Ferme Tremblaye, 78125 La Boissiere-Ecole (FR)
(72) Inventeur: CAZAJUS, Henri, 78310 Maurepas (FR); CARROUCHE, Baptiste, 78125 La Boissiere-Ecole (FR); VESCHAMBRE, Cyrille, 28130 Maintenon (FR)
(74) Mandataire: Gevers & Orès

(57) **Abrégé**

La présente invention a pour objet un procédé amélioré de méthanisation pour la production de biogaz à partir d'effluents organiques, notamment agricoles, par fermentation, le procédé comportant des étapes additionnelles conduites hors du digesteur, aération, malaxage et broyage d'une fraction de digestat et d'effluents organiques.

## Description

La présente invention a pour objet un procédé amélioré de méthanisation, c'est-à-dire de production de biogaz à partir d'effluents organiques, notamment agricoles, par fermentation.

La méthanisation est un processus de décomposition de matières organiques par des bactéries qui agissent en l'absence d'air. On nomme ce processus de décomposition « fermentation anaérobie ». Ce procédé permet de générer une énergie renouvelable, du biogaz, qui comporte entre autres du méthane (CH4, dans des proportions de 50% à 70%), et du dioxyde de carbone (CO₂) ainsi qu'un résidu organique (dit « digestat » utilisé comme fertilisant). Le biogaz peut être transformé soit en électricité, soit en gaz et en carburant pour véhicules.

Le procédé de méthanisation consiste en une fermentation d'effluents d'élevage ou de sous-produits issus de l'industrie agro-alimentaire dont le principe est le suivant : les effluents organiques sont stockés dans une cuve hermétique que l'on appelle « digesteur » dans laquelle ils sont soumis à l'action de micro-organismes (bactéries) en l'absence d'oxygène (fermentation anaérobie) pendant un temps de séjour déterminé (en général d'environ 60 jours).

La méthanisation représente un intérêt économique substantiel ; le biogaz produit peut se substituer au gaz naturel dans tous ses usages actuels : production de chaleur, production d'électricité et carburant pour véhicules, et le digestat est totalement valorisé, notamment sous forme d'engrais dont la composition est bien plus complète que celle d'un engrais chimique. Dans le cas de production d'électricité, il y a coproduction d'eau chaude de 84°C à 97°C. Elle peut donc permettre aux agriculteurs de réaliser des économies substantielles. Le biogaz peut également leur apporter un complément de revenus par la vente de l'électricité ou de gaz à des tarifs de rachat préférentiels.

La méthanisation présente également un intérêt environnemental car le biogaz est une énergie renouvelable, si sa production et son utilisation engendrent toutefois des rejets polluants dans l'atmosphère (CO₂), ceux-ci restent moins importants que ceux des énergies fossiles. Par ailleurs, le digestat est un produit fertilisant à haute valeur agronomique ; il est très facilement assimilable par les plantes de par sa teneur en ammoniac, phosphore et potassium auxquels s'ajoutent des facteurs de croissance.

La méthanisation permet également, à l'échelle locale, de supprimer le problème du stockage des matières fermentescibles (odeurs et concentration d'insectes et rongeurs).

Il existe à l'heure actuelle, deux procédés de méthanisation classiquement mis en œuvre en France ; l'un consiste à introduire des effluents organiques en continu dans un digesteur où ils sont infiniment mélangés avec le digestat déjà présent (ce procédé est dit « mésophile » ou « par voie humide », il est conduit à une température de l'ordre de 40°C) ; l'autre est réalisé par batch : les effluents organiques sont introduits dans le digesteur, la fermentation a lieu puis le digestat est récupéré par vidange du digesteur avant qu'il soit à nouveau rempli d'effluents organiques (procédé dit « thermophile » ou « par voie sèche » conduit à une température de l'ordre de 55°C). Dans ces deux procédés, la fermentation se produit par les flores spontanément présentes dans les effluents organiques.

Il existe encore d'autres méthodes de méthanisation pratiquées dans d'autres régions ; par exemple, en Allemagne, la méthanisation de type infiniment mélangée est réalisée avec de l'ensilage de maïs et nécessite l'ensemencement de micro-organismes et une parfaite maîtrise du pH. Cette pratique va à l'encontre des aspects écologiques et des aspects économiques puisqu'elle met en œuvre une matière première pouvant servir à l'alimentation animale.

Pour les raisons exposées plus tôt, il apparait utile d'optimiser la conduite de la méthanisation notamment pour en améliorer les rendements.

C'est ce à quoi est parvenue la Demanderesse en mettant au point un nouveau procédé de méthanisation par voie humide comportant plusieurs étapes additionnelles conduites hors du digesteur ou du post-digesteur lorsque le dispositif en comporte un : aération, malaxage et broyage du mélange d'une fraction de digestat et d'effluents organiques ; lors de la conduite de ces étapes additionnelles, se produit également une décantation de corps étrangers pondéreux qui peuvent être évacués régulièrement.

Plus précisément, la présente invention se rapporte à un procédé de méthanisation par voie humide d'effluents organiques dans un digesteur caractérisé en ce qu'il comporte les étapes additionnelles suivantes :
a- extraction d'une fraction du digestat présent dans le digesteur ;
b- ajout d'effluents organiques à la fraction de digestat ;
c- optionnellement, mouillage du mélange de fraction de digestat et d'effluents organiques ; le mouillage est réalisé par ajout d'un liquide aqueux, par exemple de l'eau ou du lactosérum ;
d- malaxage dans des conditions aérobies du mélange de ladite fraction de digestat, d'effluents organiques et, le cas échéant, de liquide ajouté pendant un temps déterminé ;
e- broyage du mélange obtenu à l'étape d ;
f- décantation des corps étrangers pondéreux pendant la phase de malaxage et, optionnellement, évacuation desdits corps étrangers ;
g- réintroduction dans le digesteur dudit mélange ainsi traité.

Par effluents organiques, on entend des effluents agricoles tels que du fumier issu des déjections animales, des résidus végétaux agricoles (céréales, herbes, paille...) notamment à fibres longues, du lisier, des sous-produits issus de l'industrie agro-alimentaire ou leur mélange.

L'avantage du procédé selon l'invention est qu'il ne nécessite aucun pré-traitement des effluents organiques comme un pré-broyage des brins végétaux ; en effet, il peut par exemple être mis en œuvre avec des fibres végétales longues de plusieurs dizaines de centimètres tel que le fait un Ruminant lorsqu'il ingère des tiges de graminées et les mâche.

Selon un mode de réalisation avantageux, le procédé selon l'invention est mis en œuvre avec des effluents organiques comprenant une forte proportion de fumier, c'est-à-dire jusqu'à 80% en poids de fumier, en particulier entre 60 et 80% en poids de fumier, par rapport au poids total des effluents organiques. Le fumier est un effluent organique généralement difficile à digérer dans un digesteur en ce qu'il comprend souvent des corps étrangers indésirables et nuisibles à l'installation (cailloux présents dans la paille, ficelles de bottes de paille, chutes de bâches ensilage...).

Le procédé selon l'invention peut être mis en œuvre avec tout type de fumier de ruminants comme celui de bovins mais aussi celui d'ovins ou d'équidés qui sont généralement plus secs et durs, moins dégradés et comprenant fréquemment des résidus de paille en raison de l'environnement de leur ramassage et de la grande quantité de paille utilisée pour les litières, les rendant plus difficile à méthaniser.

Le perfectionnement objet de la présente invention s'applique aux procédés de méthanisation par voie humide.

Par digesteur, on entend une cuve hermétique, généralement cylindrique, équipée d'un système de chauffage (pour le maintien d'une température comprise entre 38 et 44°C, de préférence de l'ordre de 42°C) et d'un dispositif d'agitation ; classiquement, les digesteurs sont des cuves de grande dimension, de l'ordre de 1000 à 2000 m³ voire plus, le procédé selon l'invention peut être mis en œuvre dans des digesteurs de toutes tailles.

Pour la mise en œuvre du procédé selon l'invention, la fraction du digestat contenu dans le digesteur est extraite et conduite dans un bac de malaxage (étape a du procédé), des effluents organiques à digérer (étape b du procédé) et optionnellement un liquide (étape c du procédé) sont ajoutés et le mélange est brassé en présence d'oxygène (étape d) puis broyé en présence d'oxygène (étape f du procédé).

Toutes quantités de digestat et d'effluents organiques peuvent être mises en œuvre ; typiquement, on préfèrera introduire un volume de digestat qui représente entre 60 et 80%, du volume du mélange total et le volume restant est constitué des effluents organiques, qui représente entre 20 et 40% du volume du mélange total, et de l'éventuel liquide ajouté.

Le brassage (ou malaxage) est préférentiellement mis en œuvre à l'aide de pales disposées dans le bac de malaxage.

L'aération du mélange comprenant le mélange de digestat, d'effluents organiques et de l'éventuel liquide dans le bac de malaxage est permise par une ouverture à l'air libre dudit bac de malaxage ou par injection d'oxygène dans le cas d'un bac de malaxage fermé.

Le nombre de cycles de conduite des étapes a à g, permettant l'introduction de nouvelles quantités d'effluents organiques dans le digesteur, s'ajuste en fonction du volume d'effluents organiques à traiter ; à titre d'illustration, il peut être de l'ordre de 20 cycles par 24 heures, proche de celui d'un ruminant.

Le broyage peut être réalisé par tout moyen connu de l'homme du métier ; par exemple, il peut être réalisé par la circulation du mélange obtenu à l'issue de l'étape d au travers d'une pompe hacheuse et/ou d'un broyeur, présents dans un circuit fermé positionné en sortie du bac de malaxage.

Ainsi, au cours d'un cycle de conduite des étapes a à g, le mélange de digestat, d'effluents organiques et de l'éventuel liquide subit lui-même les étapes d (malaxage) et f (broyage) de façon cyclique : le mélange est malaxé dans le bac de malaxage et circule dans le circuit fermé de broyage. De ce fait, il n'est pas nécessaire d'effectuer un pré-broyage des effluents organiques.

La durée du cycle de conduite des étapes a à g est d'au moins 1 minute, de préférence 3 minutes ; pour des raisons d'optimisation du coût énergétique du procédé, cette durée ne dépasse de préférence pas 10 minutes même si des durées plus longues ne nuiraient pas à la conduite du procédé selon l'invention.

Un liquide (par exemple de l'eau ou mieux du lactosérum) peut être ajouté au mélange de digestat et d'effluents organiques dans le bac de malaxage, cet ajout permet d'abaisser la siccité du mélange et lui confère ainsi une homogénéité et une fluidité accrues lui permettant de mieux circuler dans les conduits ; à titre indicatif, le volume du liquide ajouté à l'étape c lorsqu'elle est mise en œuvre, représente entre 15 à 20% du volume du mélange de digestat et d'effluents organiques présents dans le bac de malaxage.

A l'issue du traitement de brassage, mouillage, décantation des corps étrangers et de broyage en milieu aérobie, le mélange est réintroduit dans le digesteur tel que le fait un Ruminant lorsque le bol alimentaire retourne dans la panse après avoir été mâché.

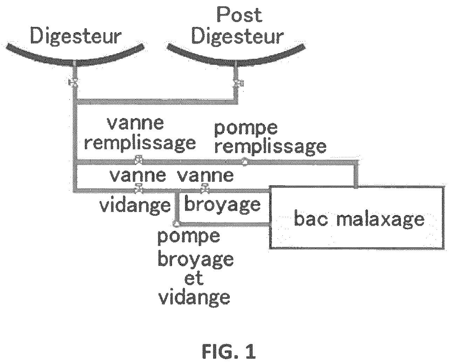

[Fig 1] représente un dispositif modulaire transportable ou adaptable à tout digesteur, approprié pour la conduite du procédé selon l'invention :
Par ce dispositif, l'extraction du digestat peut être faite soit par gravité, soit par une pompe (par ouverture de la vanne de remplissage et action de la pompe de remplissage sur la figure 1) qui l'incorpore dans le bac de malaxage ; le bac est ouvert à l'air libre pour passer d'un milieu anaérobie à un milieu aérobie tel que cela se passe dans la gueule d'un ruminant.

Le brassage durant un temps déterminé des effluents organiques introduits par des moyens adéquats avec le digestat permet la décantation des cailloux et autres corps étrangers, au fond du bac ; ils peuvent être évacués du bac de malaxage par tout moyen d'ouverture positionné sur la partie inférieure du bac.

De plus, le broyeur ou la pompe hacheuse peuvent s'ouvrir facilement pour extraire les corps étrangers non pondéreux, par exemple, les corps flottants (plastique, ficelle, etc...).

De préférence, si un liquide est introduit, il s'agit de lactosérum qui apporte des sucres (glucose notamment) nécessaires aux bactéries ainsi que des sels minéraux et autres macro éléments tels que la matière grasse et des protéines solubles.

Dans cet exemple de dispositif, la sortie du bac se fait par un orifice situé plus haut de quelques dizaines de centimètre du fond du bac afin de laisser décanter les corps étrangers apportés par les effluents organiques lors de leur ramassage. Selon la quantité de corps étrangers, le bac est vidé dès que nécessaire, ce qui évite un arrêt de production.

En fin de cycle de brassage/broyage, d'une durée déterminée comprise entre 1 minute et 10 minutes, de préférence entre 3 et 10 minutes, le mélange obtenu est réintroduit dans le digesteur (ou le post-digesteur), orienté par une électrovanne sur chacun d'eux.

Ce nouveau procédé améliore significativement la digestion de la matière organique et permet de réduire substantiellement l'énergie électrique nécessaire pour le mélange de la biomasse dans le digesteur. Ainsi, par la réintroduction du digestat traité dans le digesteur ou le post-digesteur, la production de méthane par fermentation anaérobie est plus abondante et plus rapide.

Selon un autre de ses objets, la présente invention se rapporte à un dispositif permettant la mise en œuvre du procédé de méthanisation selon l'invention.

Ce dispositif modulable et adaptable à un digesteur comprend :
- un bac de malaxage relié au digesteur ;
- un circuit de remplissage du bac de malaxage qui comprend une vanne de remplissage et une pompe de remplissage, et
- un circuit de vidange du bac de malaxage qui comprend une vanne de vidange et une pompe de vidange,
ledit bac de malaxage est également équipé d'un conduit en circuit fermé comprenant une pompe hacheuse.

Le bac de malaxage est de préférence un bac ouvert équipé d'un système d'agitation, ayant des dimensions de faible capacité de l'ordre de 50 m³ qui lui permettent d'être un module déplaçable ou adaptable à tout type de digesteur, que l'on peut aisément vidanger des corps étrangers.

Selon un mode de réalisation particulier tel que représenté à la figure 1, le conduit en circuit fermé est relié au circuit de vidange qui est le même circuit que le remplissage.

La présente invention se rapporte encore à un méthaniseur comprenant :
- un dispositif modulable et adaptable à un digesteur et
- un digesteur.

Le procédé de méthanisation amélioré selon l'invention permet une augmentation significative de la capacité de traitement d'effluents organiques. Sa mise en œuvre a permis d'augmenter le tonnage d'effluents organiques introduit dans l'installation de 2 tonnes/h à 12 tonnes/h et le ratio d'électricité produite (exprimée en kWh / tonne d'effluents) de 193% en moyenne.

Enfin, l'utilisation du cycle de malaxage/broyage dans le procédé selon l'invention a permis la suppression d'une étape de pré-broyage des effluents organiques avant introduction dans le digesteur, ce qui représente une économie significative (voir exemple 2 ci-après).

### Exemple 1 - mise en œuvre du procédé selon l'invention

A l'aide du prototype illustré à la figure 1, les opérations se font selon l'enchainement suivant :
- introduction dans le bac de malaxage d'une fraction de digestat depuis le Digesteur ou le Post-Digesteur, par gravité si la hauteur le permet, à défaut par une pompe, dite pompe en queue de cochon ;
- mélange dans le bac de malaxage à vitesse réduite pendant 8 mn pour mouiller les brins de paille issu du remplissage de 1 T de fumier par un godet ou un caisson à fond mouvant ;
- brassage pendant 8 mn à vitesse rapide des 1 T de fumier, pendant l'introduction du lactosérum ; les corps étrangers pondéreux se déposent au fond ;
- puis tout en brassant, passage par la pompe hacheuse en circuit fermé sur le bac de malaxage durant 8mn pour un débit de 125 m³/heure ;
- fermeture du circuit retour vers le bac et envoi par la pompe hacheuse, dans le Digesteur, ceci dure 10 à 15 mn selon la siccité du mélange.
Ainsi ce prototype a la capacité de passer une masse de 6T de fumier brut avec 1 m³ de lactosérum, en 30 minutes.

La mise au point de ce procédé amélioré de méthanisation permet le traitement de 12 tonnes de fumier par heure alors que le procédé « classique » par voie humide conduit avec le même digesteur ne permettait plus au bout de quelques mois, que le traitement de deux tonnes de fumier par heure (usure exagérée des organes d'introduction de fumier et de malaxage dans le digesteur).

### Exemple 2 - comparaison du rendement du procédé selon l'invention et du procédé de méthanisation par voie humide classique

L'installation, qui a été perfectionnée par le procédé selon l'invention, était précédemment utilisée, en particulier en 2015, pour conduire un procédé de méthanisation par voie humide « classique » ; c'est-à-dire qu'il comportait un digesteur dans lequel on incorporait au moyen de vis d'introduction, une masse d'effluents organiques (fumiers à fibres longues préalablement broyés) ; cette masse d'effluents organiques était ensuite fermentée en condition anaérobie dans le digesteur.

Cette installation a ensuite été équipée en 2019 d'un bac de malaxage raccordé au digesteur et a permis la mise en œuvre du procédé de l'invention ; les effluents organiques alors utilisés sont les mêmes (ils proviennent de la même exploitation) à la différence près qu'ils ne subissent pas de pré-broyage.

Le relevé des volumes d'effluents traités et de l'énergie quotidienne produite dans chacune de ces deux configurations est le suivant :

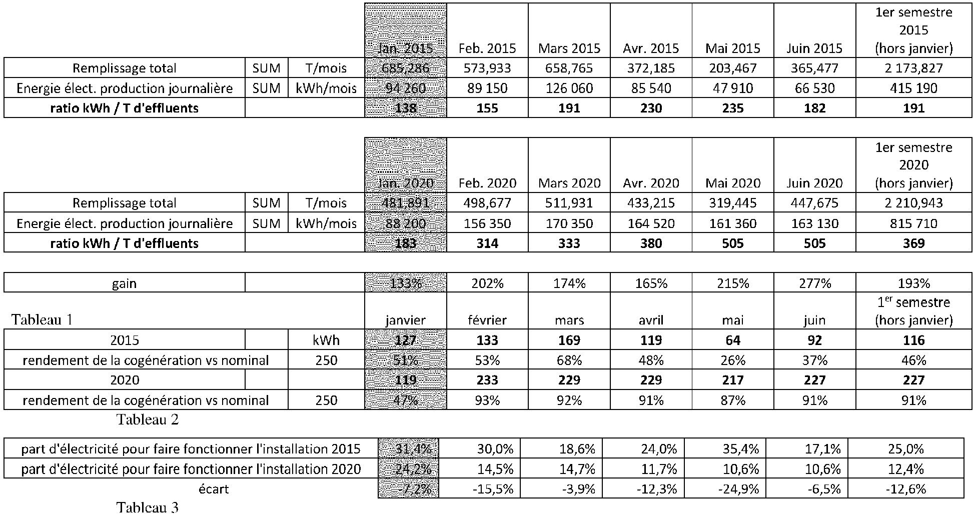

La comparaison a utilisé les données recueillies pendant l'année 2015, durant laquelle les rendements obtenus avec l'ancienne installation ont été les meilleurs, et celles recueillies pendant l'année 2020, après modification de l'installation ; les données obtenues en janvier sont mentionnées mais n'ont pas été prises en compte pour le calcul du rendement moyen car l'installation n'a fonctionné que partiellement ce mois-là. Cette comparaison montre une nette augmentation du rendement en kWh/t d'entrant : entre +133% et +277% et +193% en moyenne (voir le tableau 1).

Ce meilleur rendement s'observe également par une augmentation du rendement de cogénération qui passe de 47% à plus de 90% par rapport à sa puissance nominale de 250 kWh : voir le tableau 2.

Enfin, le rendement en kWh/T d'intrants ayant été doublé, la consommation électrique pour faire fonctionner l'installation a été divisée par 2 (voir Tableau 3) du fait de l'invention qui consiste en un procédé de "rumination".

Ces résultats montrent que la meilleure préparation de la matière augmente de façon très importante le rendement de la production de méthane.

Enfin, la suppression de l'étape de pré-broyage a généré une économie de 39 000 € sur une année complète.

## Revendications

1. Procédé de méthanisation par voie humide d'effluents organiques dans un digesteur **caractérisé en ce qu'**il comporte les étapes additionnelles suivantes :
a- extraction d'une fraction du digestat présent dans le digesteur ;
b- ajout à la fraction de digestat d'effluents organiques ;
c- optionnellement, mouillage du mélange de fraction de digestat et d'effluents organiques ;
d- malaxage dans des conditions aérobies du mélange de ladite fraction de digestat, d'effluents organiques et, le cas échéant, de liquide ajouté pendant un temps déterminé ;
e- broyage du mélange obtenu à l'étape d ;
f- décantation des corps étrangers pondéreux pendant la phase de malaxage et, optionnellement, évacuation desdits corps étrangers ;
g- réintroduction dans le digesteur dudit mélange ainsi traité.

2. Procédé de méthanisation selon la revendication 1, **caractérisé en ce que** les effluents organiques comprennent jusqu'à 80% de fumier en poids par rapport au poids total des effluents organiques.

3. Procédé de méthanisation selon la revendication 1 ou la revendication 2, **caractérisé en ce que** le liquide est du lactosérum.

4. Procédé de méthanisation selon l'une quelconque des revendications 1 à 3, **caractérisé en ce que** le volume de la fraction de digestat introduite dans le bac de malaxage représente entre 60 et 80% du volume total du mélange de digestat, d'effluents organiques et d'éventuels liquides.

5. Procédé de méthanisation selon l'une quelconque des revendications 1 à 4, **caractérisé en ce que** les conditions aérobies de l'étape d sont permises par une ouverture à l'air libre dudit bac de malaxage ou par injection d'oxygène dans le cas d'un bac de malaxage fermé.

6. Procédé de méthanisation selon l'une quelconque des revendications 1 à 5, **caractérisé en ce que** la durée totale des étapes d et f est comprise entre 3 et 10 minutes.

7. Dispositif modulable et adaptable à un digesteur comprenant :
- un bac de malaxage relié à un digesteur :
- un circuit de remplissage du bac de malaxage qui comprend une vanne de remplissage et une pompe de remplissage ;
- un circuit de vidange du bac de malaxage qui comprend une vanne de vidange et une pompe de vidange ;
ledit bac de malaxage étant équipé d'un conduit en circuit fermé comprenant une pompe hacheuse.
